# EUROPEAN PATENT APPLICATION

(11) **EP 4 368 100 A1**
(43) Date of publication of application: **15.05.2024**
(21) Application number: 22206754.8
(22) Date of filing: 10.11.2022
(51) Int. Cl.: A61B 5/021, A61B 5/00, A61B 5/022, A61B 5/0225

(54) **WEARABLE DEVICE FOR MEASURING BLOOD PRESSURE**

(71) Applicant: Hinlab, 75013 Paris (FR)
(72) Inventor: DE BEGON DE LAROUZIERE DE MONTLOSIER, Denys-Michel, 75006 Paris (FR)
(74) Representative: Icosa

(57) **Abstract**

The present invention relates to a wearable device (100) for measuring blood pressure of a subject comprising a control unit (20) including pumping means and first connection means configured to allow a fluid to flow between the pumping means and a bladder; a band device (10) configured to circle a body portion of the subject, said band device (10) comprising a bracelet (12) comprising the bladder (14); and a docking station (11) configured to removably secure the control unit (20). A first extremity (101) of the bracelet (12) is connected to a first lateral portion of the docking station (11) and a second extremity (102) of the bracelet (12) is connected to a second lateral portion of the docking station, the docking station (11) being interposed between the first extremity (101) and the second extremity (102) to close the band device (10).

## Description

### FIELD OF INVENTION

The present invention pertains to the field of devices and methods for physiological parameter estimation. In particular, the invention relates to a wearable device for measuring blood pressure and to the band device and control unit that are included in it.

### BACKGROUND OF INVENTION

Traditional sphygmomanometers comprise an inflatable cuff configured to be wrapped around an arm of a subject. The cuff may be inflated and deflated using either a manual pump or an automatized pump.

The blood pressure may be obtained by determining the systolic and diastolic pressures. To estimate the systolic pressure, the cuff has to be inflated, so as to occlude the brachial artery, while to determine the diastolic pressure, the cuff has to be deflated until blood flow in the brachial artery is restored.

Both systolic and diastolic pressures of the subject may be estimated either with an auscultatory technique or an oscillometric technique. The first technique is based on the Korotkoff sounds recorded with a stethoscope during the cuff inflations and deflations. The second technique estimates the systolic and diastolic pressures based on the amplitude of the pressure oscillations recorded within the cuff during deflation or inflation.

Several issues with traditional sphygmomanometers have arisen. Indeed, sphygmomanometers are very frequently used during auscultation at a doctor's office or in a hospital. Due to frequent manipulations, the cuff fabric tends to wear out quickly and it is necessary to change the whole device, even though the device electronics and/or pump is still functioning. This tends to be quite expensive and to create a lot of waste. Moreover, the medical staff is required to disinfect the device between to patients' auscultation. Disinfection of sphygmomanometers can be very complex because of the embedded electronics and pump that can be damaged by cleaning products or water.

A lack of adaptability to patients' different needs and morphologies has also been identified in traditional sphygmomanometers. For instance, a child has smaller limbs and a typical cuff is not adapted to the size of a child's limb. Therefore, the blood pressure measurements may be of lesser quality because the cuff is badly positioned.
The problem which the invention seeks to solve is to develop a sphygmomanometer that is more adaptable to different morphologies of patients, easier to clean and more environmentally friendly.

### INVENTION DISCLOSURE

To solve this problem, the Applicant proposes a wearable device for measuring blood pressure of a subject comprising:
- a control unit comprising:
   o a housing;
   o pumping means within said housing; and
   o first connection means in fluidic connection with the pumping means, said first connection means being configured to allow a fluid to flow between the pumping means and an external environment of the housing; and
- a band device configured to circle a body portion of the subject, said band device comprising:
   o a bracelet comprising a bladder configured to be inflated and deflated to exert a pressure on the body portion; and
   o a docking station configured to removably secure the control unit, said docking station including second connection means configured to co-operate with the first connection means to allow said pumping means to inflate or deflate the bladder.
The control unit is configured to determine blood pressure using the pressure exerted by the bladder on the body portion.
The invention is characterized in that a first extremity of the bracelet is connected to a first lateral portion of the docking station and a second extremity of the bracelet is connected to a second lateral portion of the docking station, said first lateral portion and second lateral portion facing each other. At least one of the first lateral portion and second lateral portion forms a connecting portion, said connecting portion including the second connection. As a result, the docking station is interposed between the first extremity and the second extremity of the bracelet to close the band device.

In other words, the invention proposes a device where all the electronics and pumping means are gathered and protected inside the control unit housing, while the band device only comprises the bladder. Advantageously, the control unit is removable from the band device and can be attached or detached from the band device at will.

By removing the control unit, the band device can therefore be cleaned without any risk of damaging the electronics. Moreover, when the cuff fabric is worn out, it is possible to only change the band device while keeping the control unit, which represents a real economy of resources. Therefore, the device lasts longer and is more environmentally friendly.

Advantageously, it is also possible to adapt band devices of different sizes to the control unit to better fit the patient's morphology. For instance, it is possible to use a smaller cuff for children so that the device better fits around the child's limb or bigger cuffs for overweight people. The resulting blood pressure measurement is more reliable because it circles the whole limb and because it is better positioned on said limb.

According to another embodiment, it is also possible to adapt different control units to the band device. The different control units may comprise different features such as additional sensors to measure other physiological parameters.

Therefore, the invention also relates to a kit comprising:
- a control unit, and
- a range of band devices with bracelets of different sizes to adapt to different types of body parts.

The invention also relates to a kit comprising:
- a range of control units, said control unit further comprising at least one sensor configured to measure at least one physiological parameter, and
- a band device.

The additional sensors may be an optical sensor, a temperature sensor, a heat flux sensor, a position sensor, an accelerometer, a pressure transducer, an ultrasound sensor, a gyroscopic sensor, an electrical sensor, such as for instance an electrical sensor comprising electrodes, a tomographic sensor, an acoustic sensor, a magnetometer, a humidity sensor, or even an impedance sensor. They may be used to calculate parameters such as the oxygen saturation, the heart rate, the respiratory rate, the blood pressure trend, the heart rate variability, the cardiac output, the skin temperature, the body temperature, the body composition...

In practice, the control unit housing comprises a bottom side. Said bottom side includes in particular a sensor configured to come in contact with the body portion. To better accommodate the control unit, the docking station comprises a bottom wall configured to come in contact with the skin, framed by two lateral walls comprising the second connection means. The bottom wall and lateral walls form a reception site configured to receive the control unit. The sensor can be put in contact with the skin thanks to a through hole in the bottom wall of the docking station. More specifically, the control unit housing includes a protrusion to encapsulate at least sensor, and make sure it can be applied to the skin of the patient. The protrusion containing the sensor can therefore be inserted in the through hole.

As previously explained, the cooperation between the control unit and the band device is ensured by the cooperation between the first connection means present on the control unit and the second connection means present on the docking station. On the other hand, the docking station also cooperates with the extremities of the bracelet to form the band device and allow to circle a subject's body portion. Advantageously, the second connection means may be configured to also attach at least one of the first extremity and second extremity of the bracelet to the docking station. Therefore, the second connection means have both the function of connecting the docking station with to the control unit and connecting the docking station to the bracelet, which improves the compactness of the device.

Advantageously, the second connection means include at least one sealing element comprising:
- a tube-shaped body configured to cross through the connecting portion,
- a first end configured to co-operate with the first connection means to establish a fluid-proof connection, and
- a second end configured to co-operate with the bladder to establish a fluid-proof connection.

In other words, the sealing element puts in fluidic connection the control unit with the band device and more particularly the pump with the bladder. Advantageously, the sealing element absorbs a large majority of the efforts at the connection and protects the elements included in the housing such as the pump or a pressure sensor.

In another embodiment, the connecting portion further includes at least one securing element. The securing element and the sealing element are configured to at least partially be inserted in a recess arranged in the connecting portion. This improves the compactness of the device. The securing element is configured to keep the sealing element in place and to strengthen the fluid-proof connections against impacts or wear. More precisely, the securing element may comprise an upper part and a lower part. The upper part and lower part are configured to co-operate to accommodate the tube-shaped body of the sealing element and to maintain one of the first extremity and second extremity of the bracelet at the same time. The upper part and lower part may be maintained together using fixing elements.

In practice, according to an embodiment, the bladder comprises an opening configured for the insertion of the sealing element. The second end is inserted inside this opening by deforming the flaring shape. Once inserted, the deformed second end reverts to its original flaring shape due to its high elasticity. Because the opening dimensions is smaller than the flaring shape dimensions, the latter cannot be easily removed from inside the bladder. Moreover, in the inserted position, the flaring shape is pressed against said opening to ensure fluid-proofness of the connection.

The first end of the sealing element is configured to be connected to the control unit. To that end, the first end may co-operate with several types of first connection means such as male-type first connection means or female-type first connection means.

In the first case, the first end forms inner and outer contact surfaces. The first end is arranged between the first connection means and the securing element. The first connection means come in contact with the inner contact surface of the first end, while the securing element comes in contact with the outer contact surface. The first end is therefore sandwiched and maintained into place between the securing element and the control unit, thus ensuring fluid-proofness of the connection.

In the second case, the first end forms a contact surface configured to be maintained inside said first connection means. It may be inserted in force in the connection means of the control unit. Preferably the first end is deformable to be inserted in the first connection means. Once inserted, the deformed first end reverts to its original shape due to its high elasticity. Because the first connection means dimensions are smaller than the first end dimensions, the first end cannot be easily dislodged. More precisely, the flaring shape is pressed against the first connection means to ensure fluid-proofness of the connection.

According to another embodiment, the band device includes an adjustment system configured to adapt a bracelet size to the body portion. The adjustment system may be an elastic portion, a hook and loop system or even a buckle. This buckle may be present on a lateral portion of the docking station or present on the bracelet.

According to another embodiment, the control unit includes tubing connections. For instance, the tubing connections between the pumping means and the first connection means. Advantageously, the tubing connection are made in one piece with the housing. Thus, this embodiment allows to avoid any use of flexible tubes that are more prone to tear, kinks, twists... Therefore, the process to build the control unit is faster and easier to implement.

According to another embodiment, the device includes a calibration station configured to calibrate at least one pressure sensor of the control unit. The pressure sensor is configured to determine the pressure in the bladder and exerted on the body portion. Thanks to this measurement, it is possible to determine the blood pressure of the subject. The calibration station is positioned between the docking station and the control unit. It allows to check the good-functioning of the pressure sensor and therefore the accuracy of the blood pressure measurement.

According to another aspect, the invention relates to a band device intended for use in a wearable device as described above, said band device co-operating with a control unit, said band device is configured to circle the body portion. The band device comprises:
- a bracelet comprising a bladder configured to be inflated and deflated; and
- a docking station configured to removably secure the control unit, said docking station including second connection means configured to co-operate with first connection means of the control unit, to allow said pump to inflate or deflate the bladder, and wherein a first extremity of the bracelet is connected to a first lateral portion of the docking station and a second extremity of the bracelet is connected to a second lateral portion of the docking station, said first lateral portion and second lateral portion facing each other, at least one of the first lateral portion and second lateral portion forming a connecting portion, said connecting portion including said second connection means, the docking station being interposed between the first extremity and the second extremity to close the band device.

The invention also relates to a control unit intended for use in a wearable device as previously described, said control unit co-operating with a band device, wherein the control unit comprises:
- a housing;
- pumping means within the housing; and
- first connection means in fluidic connection with the pumping means, said first connection means being configured to allow a fluid to flow between the pumping means and an external environment of the housing,
said control unit being configured to calculate a blood pressure value using the pressure exerted by the bladder on the body portion.

### BRIEF DESCRIPTION OF THE DRAWINGS

**Figure 1** is a perspective view of the device according to a first embodiment,
**Figure 2** is a top exploded view of the device from figure 1,
**Figure 3** is a bottom exploded view of the device from figure 1,
**Figure 4** is a longitudinal cross-sectional view of the device from figure 1,
**Figure 5** is a perspective view of the device according to a second embodiment where the adjustment system includes a buckle,
**Figure 6** is a perspective view of an embodiment of the docking station,
**Figure 7** is a top view of an embodiment of the control unit,
**Figure 8** is a perspective view of the control unit from figure 7,
**Figure 9** is a top view of the control unit co-operating with the docking station,
**Figure 10** is a longitudinal cross-sectional view of the control unit co-operating with the docking station from figure 5,
**Figure 11** is a perspective view of the co-operation between the docking station and sealing pieces,
**Figure 12** is a perspective view of the co-operation between the docking station and securing elements,
**Figure 13** is a perspective view of an embodiment of a sealing piece,
**Figure 14** is an exploded view of the control unit co-operation with a charging station,
**Figure 15** is a perspective view of the calibration station co-operating with the control unit and the docking station, and
**Figure 16** is a schematic front view of the calibration station from figure 15.

### DETAILED DESCRIPTION OF THE EMBODIMENTS

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The present invention relates to a wearable device **100** for measuring the vital signs and more particularly the blood pressure of a subject.

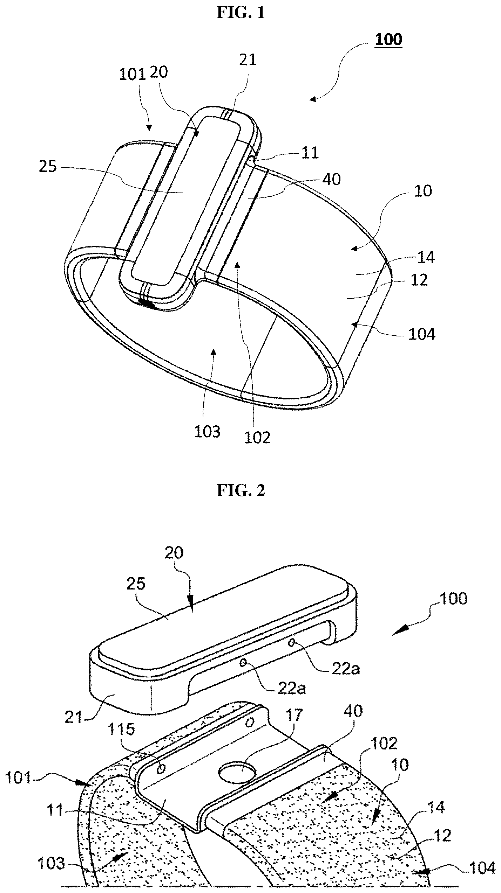

As illustrated on figures 1 to 5, the device **100** comprises a control unit **20** cooperating with a band device **10.** The control unit **20** comprises all the electronics and pumping means gathered and protected inside a housing **21,** while the band device **10** only comprises the bladder **14.** The control unit **20** is removable from the band device **10** and can be attached or detached from the band device **10** at will.

### CONTROL UNIT

The control unit **20** comprises a housing **21** with a substantially parallelepiped shape. Advantageously, the housing **21** has a length for example comprised between 5 and 10 cm, a width for example comprised between 2 and 5 cm and a thickness for example comprised between 0.5 and 3 cm. Corners of the housing **21** may have a more rounded shape for aesthetic purpose. The housing **21** may be made of any kind of solid or flexible material, such as: polypropylene, ABS, PVC, Polyamide, rubber, resin, silicone, urethane, nylon, glass such as Gorilla^{®}, or titanium.

The control unit **20** may be opened thanks to a removable lid, not represented in the figures. The removable lid may be the upper or lower wall of the housing **21.** In one embodiment, the screen is removable and allows access to the inside of the housing **21.**

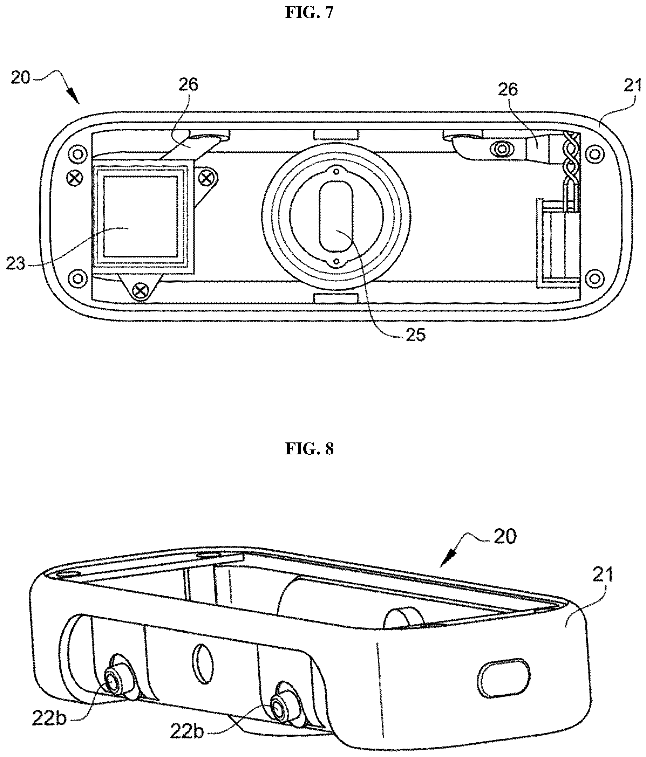

As illustrated on figures 7 and 8, the inside of the housing **21** is hollow and contains the electronics and pumping means.

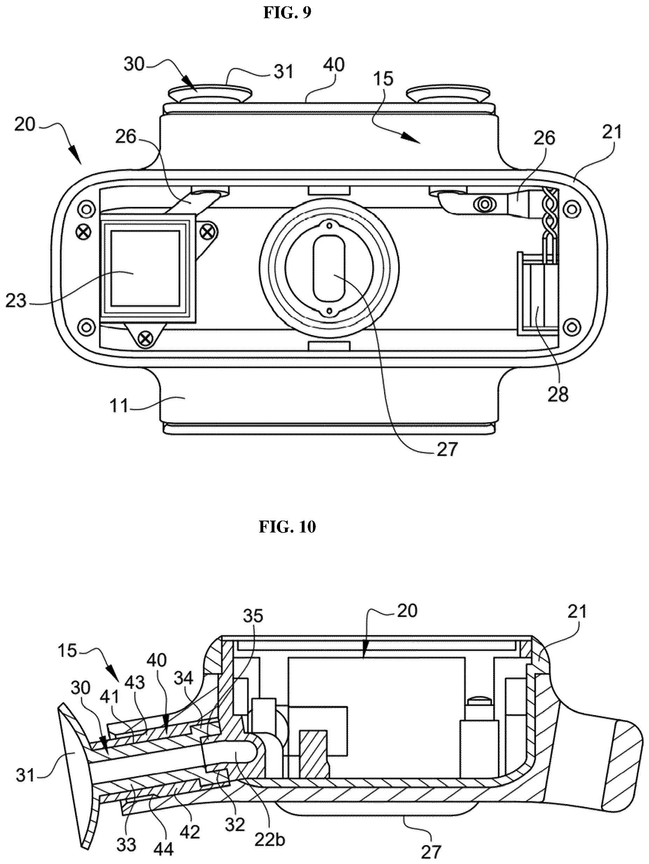

The pumping means comprise a pump **23,** represented on figure 9, that may be a rolling air pump, a piezo electric pump, a resonant gas pump, a diaphragm/membrane gas pump, and the like.

The pump **23** is in charge of putting a fluid in motion. Preferably, the fluid is air. The pump **23** is in fluidic connection with first connection means **22a, 22b.** The fluid may flow in a first direction, from the first connection means **22a, 22b** toward the pump **23** and in a second direction, from the pump **23** toward the first connection means **22a, 22b.**

In practice, the first connection means **22a, 22b** include at least an opening localized in a wall of said housing **21** and configured for the passage of the fluid between the inside of the control unit **20** and the external environment. For instance, the first connection means may be male-type first connection means **22b** as illustrated on figure 8, meaning that they may protrude from the control unit **20.** For instance, male-type first connection means **22b** present a tube-like shape with a length comprised between 0.3 and 1 cm and an external diameter comprised between 0.1 to 0.5 cm. Alternatively, the first connection means may be female-type first connection means **22a,** as illustrated on figure 2, meaning that they can either be a mere opening or protrude toward the inside of the control unit **20.**

The pump **23** is connected to the first connection means **22a 22b** with a network of tubing connections **26.** Said tubing connections **26** may be flexible or rigid.

Moreover, the network of tubing connections **26** may be built in one piece, for instance by molding or 3D printing. The resulting piece may be fixed to the housing **21** using connection means. For instance, a frame connects the network of tubing connections **26** and said frame may be clipped on the housing **21** using a plug and socket system.

According to an embodiment, the first connection means **22a, 22b** include two elements of connection: a first element configured to let fluid enter the housing **21** and a second element configured to let fluid exit the housing **21.** The pump **23** may be connected to both elements with a dedicated arrangement of tubing connections **26.** The pump **23** is therefore configured to pump the fluid outside of the housing **21** through the first elements of connection and to pump the fluid inside the housing **21,** through the second element. Alternatively, there is only one element of connection, through which the pump **23** may pump-in and out the fluid.

Both elements of the first connection means **22a, 22b** may be positioned on the same side of the housing **21** or alternatively, a first element may be positioned on a first lateral side of the housing **21** and the other element may be positioned on the opposite lateral side.

At least one valve, not represented in the figures, may also be included in the pumping means to control the passage of fluid through the network of tubing connections **26,** for instance by opening, closing, or partially obstructing at least one of the tubing connections **26.** Alternatively, a protection system could be included with a second valve in case of failure of the first valve, to ensure that there is no long-term arm compression of the patient. The valve may be physically separated from the pump **23** or integrated in the pump **23.** For instance, the pump **23** may comprise one or more blowers, which allows to discharge air, thereby providing a passive valve function.

In another embodiment, the first element of the first connection means **22a, 22b** may be connected to the pump **23** and the other element may be connected to the valve.

As an alternative to the pump **23,** the pumping means may comprise two distinct components, one of which is configured to pump the fluid in, and the other one configured to pump the fluid out.

The control unit **20** may also comprise a power supply device, not represented in the figures, configured to supply power to the components of the control unit **20.** Said power supply device may comprise a built-in battery and/or removable battery. In one embodiment, the removable battery is rechargeable. In another embodiment, the built-in battery has capacity comprised between 10 mAh and 300 mAh, preferably between 30 mAh and 100 mAh. This embodiment ensures that the built-in battery duration is long enough to ensure the continuity of the functions whilst the removable battery is not in place, for instance while the removable battery is being replaced or recharged. For instance, the continuity of the functions may be ensured for at least 30 minutes; preferably for at least 40 minutes, 50 minutes or 60 minutes.

The device **100** of the invention may be configured to work for the auscultatory technique or the oscillometric technique.

The first technique is based on the Korotkoff sounds recorded during the cuff inflations and deflations. In that case, the control unit **20** may not include any other feature. The medical staff may parameter the control unit **20** to inflate and deflate the bladder **14** while listening to the Korotkoff sounds with a stethoscope. Alternatively, the device **100** may include a sensor configured to automatically record the Korotkoff sounds. Such sensor may be a PPG sensor for instance.

The second technique estimates the systolic and diastolic pressures based on the amplitude of the pressure oscillations recorded within the cuff during deflation or inflation. In that case, the control unit **20** preferably includes at least one pressure sensor **28.** This allows to monitor the pressure in the bladder **14** and to obtain the blood pressure of the subject therefrom, via an oscillometric technique or "cuff-based" technique. More precisely, oscillometry is a technology based on the principle of occluding blood flow within the patient's limb with the inflatable cuff. Unlike the auscultation method, it measures the pulses transduced through the inflatable cuff, and exploits the fact that the pulse amplitude is modulated by the difference between the mean arterial pressure and the applied pressure in the cuff. The pulses amplitude increases when the applied pressure is between the patient's diastolic blood pressure and the mean arterial pressure, while the pulses amplitude decreases when the applied pressure is between the mean arterial pressure and the systolic blood pressure. Variation of pulses amplitude with the applied pressure yields a quasi-symmetric waveform from which the systolic and diastolic blood pressures can be determined using empirical formulas. Advantageously, the oscillometric technique allows to independently measure systolic and diastolic blood pressures. Moreover, the oscillometric technique provides an absolute blood pressure value.

In one embodiment, the device measures pressure during a controlled deflation of the bladder. Preferably, in another embodiment, the device measures blood pressure during the inflation of the bladder. This method ensures the patient's comfort because of the shorter occlusion time of the artery. It also has a reduced battery consumption.

The pressure sensor **28** may be of any type, for instance it may be a piezoresistive pressure sensor.

According to an embodiment, the pressure sensor **28** is connected to one of the first connection means **22a, 22b,** while the pump **23** is connected to the other connection means **22a, 22b.** This configuration allows to get better pressure measurements in particular when the pump **23** is activated during the blood pressure measurement.

In another embodiment, the pump **23** and the pressure sensor **28** are connected to the same first connection means **22a, 22b.**Advantageously, a protection system could be included with a second pressure sensor in case of failure of the first pressure sensor, to ensure that the patient's limb is not compressed for an extended period of time.

In more advanced sphygmomanometers, the blood pressure value may be obtained using several techniques at the same time. For instance, a first blood pressure value may be obtained using a cuff-based technique and a second blood pressure value may be obtained using an optical sensor **27.** Therefore, the control unit **20** may also comprise at least one optical sensor **27.** The optical sensor **27** is arranged so that it is in close contact with the subject's skin to allows to acquire a signal at an elevated acquisition frequency, which is suitable for a continuous or regular longitudinal monitoring of the subject's blood pressure.

Preferably, the optical sensor **27** is a photoplethysmographic (PPG) sensor comprising a light source and a light detector, more preferably a single-wavelength PPG sensor. For instance, the light source may be an infrared LED. In one embodiment, the light source emits an infrared radiation from about 700 nm to about 1000 nm, preferably from about 900 nm to about 980 nm. In one preferred embodiment, the light source is configured to emit an infrared radiation of about 940 nm. The infrared radiation wavelengths herein mentioned preferably are the centroid wavelengths or the peak wavelengths of the light emitters at their operating temperature. The device may comprise several optical sensors, which may be structurally independent or embedded within a sensor array. In one particular example, the device comprises at least three optical sensors and preferably: at least one infrared LED, at least one green LED, at least one red LED. Preferably, said infrared LED, green LED and red LED are configured to emit a radiation having a radiation wavelength of 940 nm, 536 nm, and 655nm, respectively. Advantageously, the optical sensor may further comprise a light filtering element configured to minimize the intensity of the light reflected from the skin surface without affecting the light reflected by subcutaneous tissues. The light filter element may comprise a first sheet of polarized glass placed on the light source of the optical sensor and/or a second sheet of polarized glass placed on the light detector of the optical sensor.

Preferably, the housing includes an opening in its inferior wall to allow the optical sensor to take its measurements. The opening is preferably made with a circular shape with a diameter for example comprised between 0.5 and 3 cm. The optical sensor **27** may protrude through the opening to come in close contact with the subject's skin.

Additionally, the control unit **20** may include other sensors such as a temperature sensor, a heat flux sensor, a position sensor, an accelerometer, a pressure transducer, an ultrasound sensor, an accelerometer, a gyroscopic sensor, an electrical sensor, such as for instance an electrical sensor comprising electrodes, a tomographic sensor, an acoustic sensor, a magnetometer, a humidity sensor, or even an impedance sensor. They may be used to calculate parameters such as the oxygen saturation, the heart rate, the respiratory rate, the blood pressure trend, the heart rate variability, the cardiac output, the skin temperature, the body temperature, or even the body composition.

As illustrated in figures 1 or 2, a top external part of the housing **21** may be covered by a screen **25** to allow visualization of information such as the subject's blood pressure or other vital signs measured by the device **100.** The screen **25** may be a liquid-crystal display (LCD), organic light emitting diode (OLED) display, light emitting diode (LED) display, a e-ink display, or the like. The housing **21** may also include button and/or switches on its external surface to power on/off the device **100** or to configure the device **100.** Alternatively, the screen **25** may be touch-sensitive.

### BAND DEVICE

The band device **10** is configured to be worn around a body portion of the subject, such as a limb or the torso and preferably around an arm of the subject, and more particularly around the upper arm. When worn around the upper arm, the device **100** is less sensitive to motion artifacts compared to wearable devices worn around the wrist or a finger. Moreover, the upper arm is characterized by elevated vascularization, and presence of main arteries such as the brachial artery. Therefore, the blood pressure measured with the present device **100** is indicative of the blood pressure in the arm arteries, such as the brachial or humeral artery, which is close to the blood pressure in the aorta. This allows to provide a highly accurate and non-invasive blood pressure estimation.

As illustrated on figures 1 to 4, the band device **10** comprises a docking station **11** connected to a bracelet **12** including a bladder **14.**

The bracelet **12** may be obtained using two bands of fabric sawn together to form a sleeve where the bladder **14** can be fitted. The bracelet fabric may be a nylon or synthetic fiber fabric, which protects the bladder **14** from cuts during use by on-scene rescue technicians and reduces patient discomfort. The bracelet fabric is preferably very flexible to adapt to the bladder **14** inflations and deflations, durable and waterproof to resist numerous uses.

Alternatively, the bladder **14** may be directly exposed and not covered with a protective sleeve. In that case, the bladder **14** is preferably made of a thicker material to withstand wear.

The bracelet **12** has preferably a length for example comprised between 20 cm and 42 cm, preferably between 20 cm and 36 cm or between 20 cm and 30 cm. The bracelet **12** has a width that may be comprised between 5 cm and 16 cm, preferably between 9 cm and 14 cm.

An inner surface **103** and an outer surface **104** of the bracelet **12** may be defined, the inner surface **103** being configured to be in contact with the skin of the subject when the device **100** is worn. A first extremity **101** of the bracelet **12** may be permanently connected to the docking station **11** while the second extremity **102** may be used to adjust the size of the bracelet **12** to the body portion size.

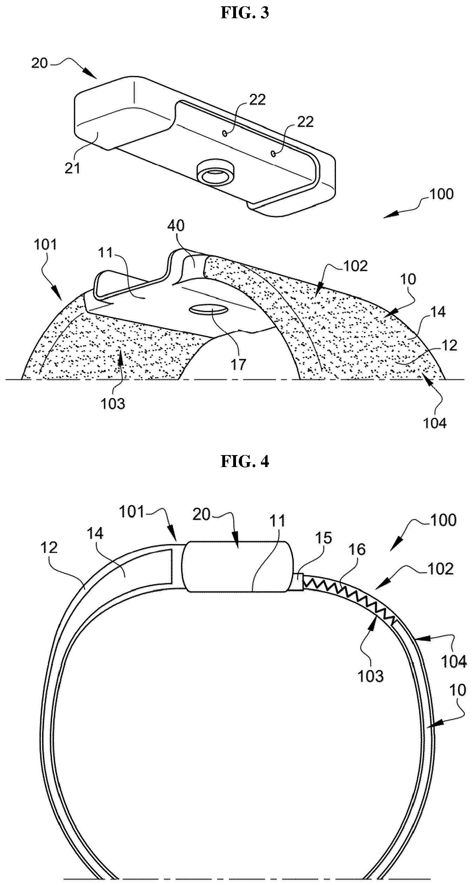

In a first embodiment, the second extremity **102** is also permanently connected to the docking station **11** and the adjustment system includes an elastic portion **16** such as illustrated in figure 4. Thus, the device **100** may be put on or removed by deforming the elastic portion **16.**

In another embodiment, the second extremity **102** may be removably connected to the docking station **11,** thus allowing to open the bracelet **12** to put on or remove the device **100.**

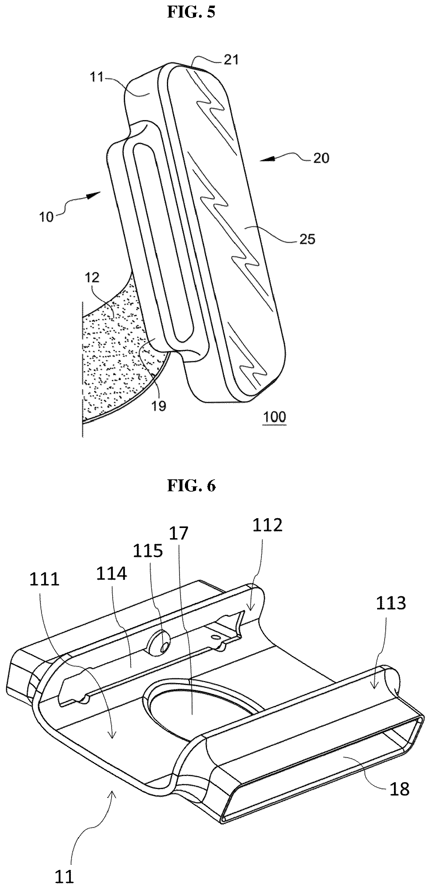

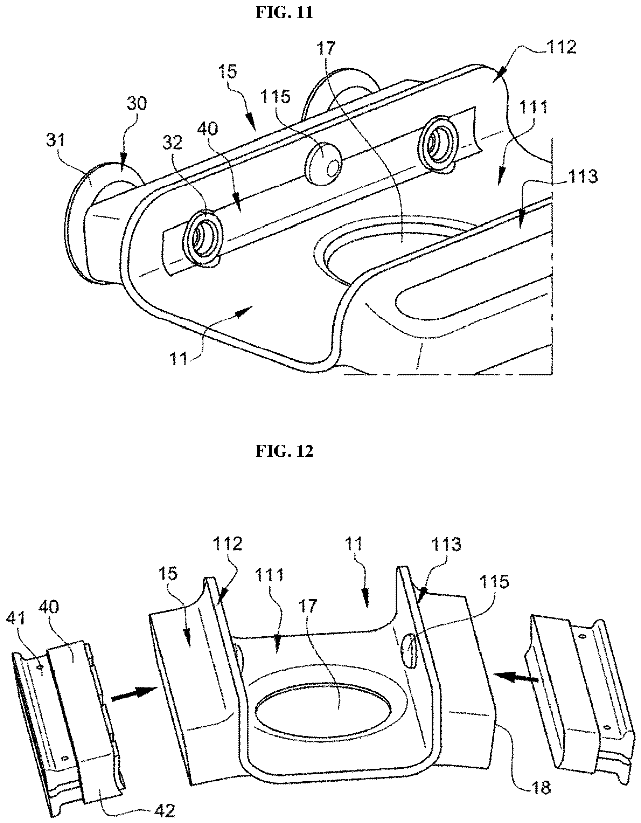

As illustrated on figure 5 and figure 11, the adjustment system may include a buckle **19** formed on a lateral portion of the docking station **11** and fixation means such as a hook and loop system or snap fasteners on the second extremity **102** of the bracelet **12.** The second extremity **102** can be threaded through the buckle **19** and fastened on itself to adjust the size of the bracelet **12.**

As aforementioned, a bladder **14** is included between the inner surface **103** and outer surface **104.** The bladder **14** is inflatable to a pressure that is high enough to uniformly restrict the blood flow in the subject's arm around which the bracelet **10** is worn. In other words, the bladder **14** may be inflatable up to a maximum inflation pressure which is superior to the pressure needed to occlude the brachial artery. Said maximum inflation pressure may be equal to 250 mm Hg, preferably 260 mm Hg, 270 mm Hg or 280 mm Hg. In some cases, it may be desirable to provide a bladder **14** inflatable up to a pressure of 300 mm Hg.

According to one embodiment illustrated on figure 4, the inflatable bladder **14** may be made of a single inflatable chamber that does not extend throughout the length of the bracelet **12.** For instance, the inflatable bladder **14** may extend for a length between 20 and 42 cm preferably between 22 cm and 36 cm., so as to cover for instance 80% of the wearer's arm. Alternatively, the bladder **14** may extend throughout the length of the bracelet **12.** Alternatively, the bladder **14** may contain several inflatable chambers distributed along the bracelet **12.**

The bladder **14** may be made of resin, rubber, polyester, synthetic fiber, or a combination thereof. Preferably, the bladder **14** is made of plastic fibers, TPU, PVC, PA or a combination thereof. In some embodiments, the bladder **14** may be made of coated fabrics such as Nylon^{™}.

The bladder **14** includes an opening in which a tubing connection may be fitted.

As previously described, the bracelet **12** only partially circles the body portion. The remaining portion is completed by the presence of the docking station **11.**

As illustrated on figure 6, the docking station **11** comprises a bottom wall **111** configured to come in contact with the skin, framed by two lateral walls **112** and **113,** forming together a reception site configured to receive the control unit **20.** The first lateral wall **112** defines a first lateral portion and the second lateral wall **113** defines a second lateral portion.

For instance, the reception site may have a complementary shape to the control unit **20.** Therefore, the reception site may have a parallelepiped shape with a length for example comprised between 5 and 10 cm, a width for example comprised between 2 and 5 cm and a thickness for example comprised between 0.5 and 3 cm.

### COOPERATION BETWEEN THE CONTROL UNIT AND THE BAND DEVICE

The control unit **20** may be plugged and unplugged on the band device **10** at will. To that end, the control unit **20** may be fitted inside the reception site of the docking station **11** in several ways. For instance, the control unit **20** may be inserted by a movement from top down. Alternatively, the control unit **20** may be inserted by a sliding movement from one side to the other. In that case, the control unit **20** may include a rail cooperating with a slide on the docking station side walls **112, 113.**

Once installed on the docking station **11,** the control unit **20** may either extend over the ends of the docking station **11** or be completely enclosed inside the reception so that it is protected from chocs and cannot be easily dislodged.

The control unit **20** may be maintained in the reception site using fixing means, such as deformable ergots **115,** as illustrated in figure 6, or a plug and socket system. Alternatively, the fixing means is a system of clip with male ergots on the reception site and female ergots on the control unit **20.** Moreover, the control unit **20** may present slots to receive such fixing means.

Advantageously, the docking station **11** comprises a through hole **17** in its bottom wall **111.** The through hole **17** is configured to allow insertion of a sensor such as an optical sensor **25** of the control unit **20.** Preferably, the through hole **17** is made with a circular shape with a diameter comprised between 0.5 and 3 cm. The through hole **17** in the docking station bottom wall **111** is configured to be aligned with the opening of the control unit **20** to allow the optical sensor **25** to protrude through the through hole **17** and come in close contact with the subject's skin.

The connection between the control unit **20** and the band device **10** and more precisely between the pump **23** and the bladder **14** is ensured by the association of several elements. In the end, the first connection means **22a, 22b** from the control unit **20** have to be put in fluid-proof contact with the second connection means **15** of the docking station **11.**

For that purpose, the docking station **11** comprises a connecting portion corresponding to at least one of the first lateral portion and second lateral portion of the docking station **11,** said connecting portion including the second connection means **15.** On figure 6, the connecting portion corresponds to the first lateral wall **112** in which an opening **114** is created. Moreover, the external surface of the first lateral wall **112** forms a recess **18.** This arrangement is configured to receive a first securing element **40** and at least one sealing element **30.**

### Securing element

The securing element **40** is illustrated in figures 9 to 12. The securing element **40** is configured to be fitted in the recess **18.** In the illustrated embodiment, the securing element **40** has a parallelepiped shape with a length for example comprised between 5 and 15 cm, a width for example comprised between 2 and 5 cm and thickness for example comprised between 0.5 and 1.5 cm. It comprises at least one opening that crosses along its entire length, the opening being configured to receive the body of a sealing element **30.** Advantageously, the securing element **40** is made of two parts: an upper part **41** and a lower part **42.** Both parts cooperate to form the opening configured to receive the body of the sealing element **30.** For instance, the upper part **41** and lower part **42** may comprise a slot crossing along their respective length, so that when the two parts are assembled, the opening configured to receive the sealing element **30** is formed. The opening has preferably a cylindrical shape and the slots may have a hemi-cylindrical shape, so that when the upper part **41** and a lower part **42** are assembled, the cylindrical opening is formed.

The securing element **40** may have two of such openings, in order to fit two sealing elements **30.** One sealing element **30** being configured for the passage of a fluid in a first direction and the other sealing element being configured for the passage of a fluid in the opposite direction. Alternatively, the securing element **40** may only have one opening, in order to fit a single sealing element **30** configured for the passage of a fluid in both directions.

The upper part **41** and lower part **42** may be fixed together using fixing elements such as screws and bolts.

The securing element **40** may present notches **43, 44** on the outer face of the upper part **41** and lower part **42.** The notches **43, 44** are designed to create an insertion recess configured for the insertion of the bracelet inner surface and an outer surface fabric.

### Sealing elements

The sealing element **30** is preferably made of any material exhibiting elastic or rubber-like properties, such as polymers, neoprene, ethylene-vinyl acetate, nitrile, Closed Cell Silicone Sponge, ABS, polycarbonate (Lexan, Makrolon), polyethylene (LLDPE, LDPE, MDPE, HDPE, HMW-HDPE), polypropylene (homopolymer, copolymer), polystyrene (HIPS, crystal styrene), polyurethane (polyester, polyether), PVC (rigid, flex), synthetic rubber (TPV, TPU, TPE, OBC). Therefore, the sealing element **30** can easily be deformed to be inserted in openings or to fit around connection elements.

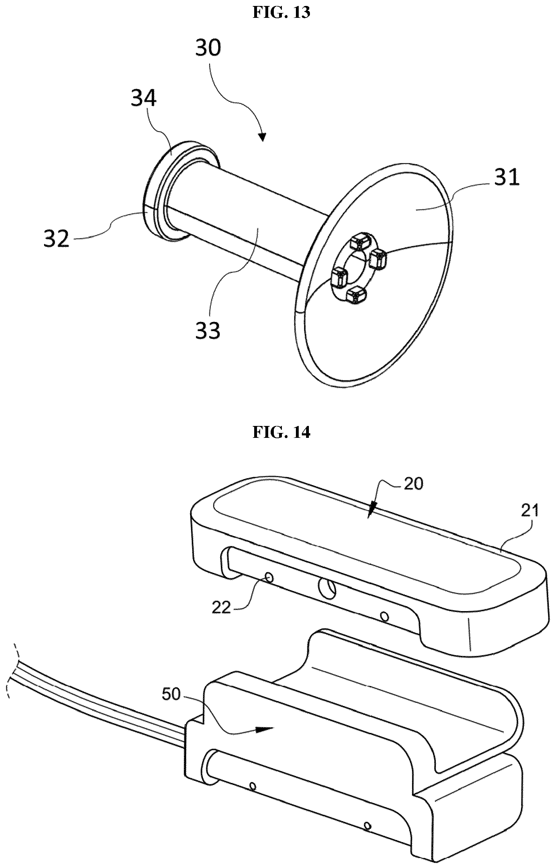

As illustrated in figure 13, the sealing element **30** comprises a tube-shaped body **33** and two ends. The body **33** is hollow and can be crossed through by a fluid. The external diameter of the body **33** is comprised for example between 0.1 and 1 cm and the internal diameter of the body **33** is comprised for example between 0.05 and 0.8 cm. The length of the whole sealing element **30** is comprised for example between 2 and 5 cm.

The first end **31** of the sealing element **30** may have a flaring shape, meaning that the diameter of the sealing element **30** increases at its first end **31.** For instance, the flaring shape corresponds to a shoulder, meaning that the diameter increases radially such as illustrated in figure 13. Hence, the external diameter of the first end **31** is comprised for example between 0.2 and 1 cm and the internal diameter of the tube is comprised for example between 0.02 and 0.8 cm. Alternatively, the diameter may increase gradually, thus forming a paraboloid shape. Either way, the first end **31** forms contact surfaces **35, 36** configured to guarantee fluid-proofness of the connection.

For instance, the first end illustrated in figure 10 is in the form of a shoulder. When the control unit **20** is fitted in the docking station reception site, the male-type first connection means **22b** crosses through the opening **114** in the lateral wall **113.** The cooperation between the male-type first connection means **22b** and the first end **31** is obtained by inserting in force the male-type first connection means **22b** inside the first end **31** opening. The inner diameter of the first end **31** forms an inner contact surface **35** for the male-type first connection means **22b,** while the transversal part of the shoulder portion forms an outer surface **36.** This outer surface **36** is configured to come in contact with a side wall of the securing element **40.** The securing element **40** maintains the first end **31** around the male-type first connection mean **22b** of the control unit **20.** The first end **31** is therefore sandwiched and maintained into place between the securing element **40** and the control unit **20,** thus ensuring fluid-proofness of the connection.

According to another example, the first connection means is a female-type first connection mean **22a** such as illustrated in figure 2. In that case, the first end, not represented in the figures, may have a flaring shape in the form of a paraboloid where the apex is directed toward the extremal part of the first end, while the base is directed toward the second end **32.** The cooperation between the female-type first connection means **22a** and the first end is obtained by inserting in force the first end inside the female-type first connection mean **22a** opening. When inserted, the paraboloid shape is deformed to fit in the opening. Once the base of the paraboloid has crossed the opening, it can deploy back to its original shape. Therefore, the base of the paraboloid forms a contact surface of bigger diameter than the opening diameter. Said base can be pressed against the opening of the female-type first connection means **22a,** thus ensuring fluid-proofness of the connection. Therefore, the fluid can transit between the network of tubing connections **26** and the body **33** without any leak.

The second end **32** of the sealing element **30** also has a flaring shape, meaning that the diameter of the sealing element **30** increases at its second end **32.** Advantageously, the diameter of the second end **32** increases gradually, thus forming a paraboloid shape where the base is directed toward the extremal part of the second end **32.** The diameter may for instance increase from 0.2 cm to 3 cm.

In use, the second end **32** is inserted inside the bladder **14** by deforming the flaring shape. Once inserted, the deformed second end **32** reverts to its original flaring shape and can be pressed against the opening of the bladder **14** to ensure fluid-proofness of the connection. Alternatively, screws or a clip system may be used to ensure fluid-proofness of the connection.

When mounting the device **100,** the sealing elements **30** are first fitted inside said securing element **40** so that both the first end **32** and the second end **31** extend over the edges of the securing element **40.** Said sealing **30** element can either be inserted in force inside the securing element **40** opening by a deformation of its body or the upper part 41 and lower part **42** may be closed around the sealing element body **30.** Then the second end **31** is inserted inside the bladder **14.** The inner surface **103** and outer surface **104** fabric of the bracelet **12** are then fitted against the sides of the recess **18** and the securing element **40** is inserted in the recess **18,** as illustrated in figure 12, on one side of the docking station **11,** thus blocking the inner surface **103** and an outer surface **104** fabric of the bracelet **12** in the notches **43, 44,** between the recess walls and the securing element **40.**

On the other side of the docking station **11,** the inner surface **103** and outer surface **104** fabric of the opposite end of the bracelet **12** may also be fitted against the walls of a second recess **18.** A second securing element **40,** with no sealing element **30** inside, may be inserted in said recess **18,** thus blocking the inner surface **103** and an outer surface **104** fabric.

In another embodiment, the other side of the docking station **11** has no recess **18** and a buckle **19** is present instead, thus allowing to attach the other end of the bracelet **12.**

Lastly, the control unit **20** is fitted inside the reception site of the docking station **11,** either by first inserting the male-type first connection means **22b** in the first end **31** of the sealing element **30** or by plugging the first end **31** inside the female-type first connection means **22a.**

### CALIBRATION STATION

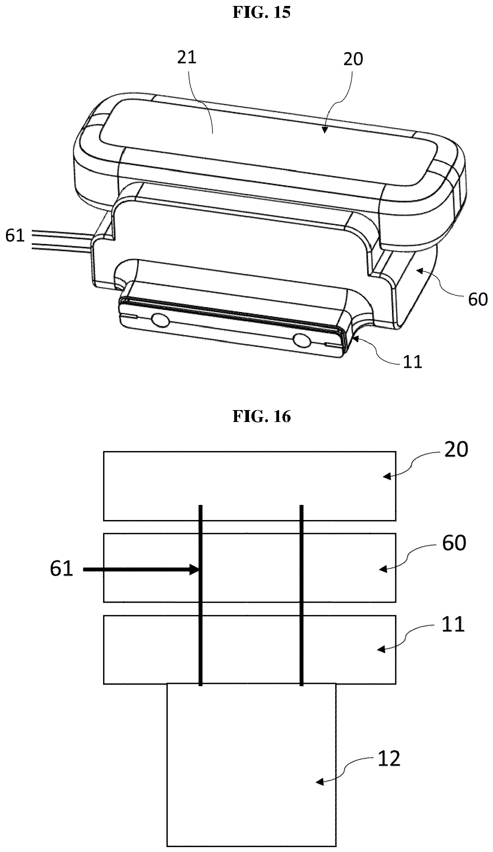

Advantageously, the device **100** includes a calibration station **60,** as illustrated in figures 15 and 16. The calibration station **60** is used to test the device **100** and to verify if it meets safety standards such as the ISO standards IEC 80601-2-30 on medical electrical equipment.

For this purpose, the calibration station **60** may check different aspects of the device **100.** For instance, it checks if the pressure sensor **28** is accurate using a simulator **61.** It checks if there are any leaks problems in the device **100.** It performs "static pressure" tests. It may also check that the safety modes work well. Such safety modes may be an automatic deflation in the event of overpressure. For instance, the safety mode may prevent the pressure in the bladder **14** from exceeding a maximum value such as the one specified in the ISO standards IEC 80601-2-30, clause 201.12.1.104, which is of 300mmHg, by more than 10 %, for more than 3 seconds. As another example, the safety mode may prevent the pressure in the bladder **14** from exceeding a maximum value such as the one specified in the ISO standard 201.12.1.104 for more than 15 seconds. When activated, the safety mode deflates the bladder **14** within 30 seconds to a pressure value inferior to 15 mmHg.

As illustrated in figure 16, the calibration station **60** may be interposed between the control unit **20** and the docking station **11.** The calibration station **60** may comprise an upper side framed by two lateral walls comprising connection means configured to cooperate with the first connection means **22a, 22b.** The upper side and lateral walls form a reception site configured to receive the control unit **20.**

The calibration station **60** may also include a lower side comprising connection means configured to fit in the docking station **11** reception site and to cooperate with the second connection means **15.** Therefore, when the calibration station **60** is interposed between the control unit **20** and the docking station **11,** it ensures connection of the pumping means with the bladder **14,** through the calibration station **60,** thus making it possible to perform the different tests.

To conclude, the invention allows to obtain a modular device with an easy-to-use cooperation system between the different available control units and different band devices. The device is very adaptable to patients with different needs and morphologies.

### KIT

The device **100** is configured so that it is possible to connect a range of different control unit **20** with different features on the band device **10.** In the same manner, the device is also configured to allow the connection of band devices **10** with different features to the control unit **20.**

For instance, it is possible to have a kit with a control unit **20** and several band devices **10** with different sizes of bracelet **12.** For instance, a first band device **10** may have a bracelet **12** with a length of 15 cm dedicated to children, a bracelet **12** with a length of 25 cm and a bracelet **12** with a length of 35 cm.

As another example, the kit may comprise a band device **10** with several control units **20.** The control unit **20** may be chosen depending on the patient's need. A first control unit **20** may include only sensors for taking a blood pressure value. A second control unit **20** may include a position sensor and/or an accelerometer to also detect the patient's movements during the day and correlate the with the blood pressure variations. A third control unit **20** may include additional sensors to measure other vital signs such as the oxygen saturation, the heart rate, the respiratory rate and the body temperature for a comprehensive monitoring of the patient.

Such as illustrated in figure 14, the kit may also include a charging station **50** to charge the control unit **20** battery. The control unit **20** may cooperate with the charging station **50** in the same way than with the band device **10.** As such, the control unit **20** may be fitted inside the reception site of the charging station **50** by a movement from top down. Alternatively, the control unit **20** may be inserted by a sliding movement from one side to the other. In that case, the control unit **20** may include a rail cooperating with a slide on the charging station side walls.

## Claims

1. A wearable device (100) for measuring blood pressure of a subject comprising:
- a control unit (20) comprising:
o a housing (21);
o pumping means (23) within said housing (21); and
o first connection means (22a, 22b) in fluidic connection with the pumping means (23), said first connection means (22a, 22b) being configured to allow a fluid to flow between the pumping means (23) and an external environment of the housing (21);
- a band device (10) configured to circle a body portion of the subject, said band device (10) comprising:
o a bracelet (12) comprising a bladder (14) configured to be inflated and deflated to exert a pressure on the body portion; and
o a docking station (11) configured to removably secure the control unit (20), said docking station (11) including second connection means (15) configured to co-operate with the first connection means (22a, 22b) to allow said pumping means (23) to inflate or deflate the bladder (14),
***wherein*** said control unit (21) is configured to determine blood pressure using the pressure exerted by the bladder (14) on the body portion, ***characterized in that*** a first extremity (101) of the bracelet (12) is connected to a first lateral portion of the docking station (11) and a second extremity (102) of the bracelet (12) is connected to a second lateral portion of the docking station, said first lateral portion and second lateral portion facing each other, at least one of the first lateral portion and second lateral portion forming a connecting portion, said connecting portion including said second connection means (15), the docking station (11) being interposed between the first extremity (101) and the second extremity (102) to close the band device (10).

2. Device according to claim 1, ***wherein*** said second connection means (15) are further configured to attach at least one of the first extremity (101) and second extremity (102) of the bracelet (12) to the docking station (11).

3. Device according to claim 1 or 2, ***wherein*** said second connection means (15) include at least one sealing element (30) comprising:
o a tube-shaped body (33) configured to cross through the connecting portion;
o a first end (32) configured to co-operate with the first connection means (22a, 22b) to establish a fluid-proof connection; and
o a second end (31) configured to co-operate with the bladder (14) to establish a fluid-proof connection.

4. Device according to claim 3, ***wherein*** said connecting portion further includes at least one securing element (40), said securing element (40) and said sealing element (30) being configured to at least partially be inserted in a recess (18) arranged in the connecting portion.

5. Device according to claim 4, ***wherein*** said securing element (40) comprises an upper part (41) and a lower part (42), said upper part (41) and lower part (42) being configured to co-operate to accommodate the tube-shaped body (33) of the sealing element (30) and to maintain one of the first extremity (101) and second extremity (102).

6. Device according to any of claims 3 to 5, ***wherein*** the first end (32) forms inner and outer contact surfaces, said first end (32) being arranged between the first connection means (22a, 22b) and the securing element (40), the first connection means (22a, 22b) coming in contact with the inner contact surface and the securing element coming in contact with the outer contact surface.

7. Device according to any of claims 3 to 5, ***wherein*** said first end (32) forms a contact surface configured to be maintained inside said first connection means.

8. Device according to any of claim 1 to 7, ***wherein*** said band device (10) includes an adjustment system (16) configured to adapt a bracelet size to the body portion.

9. Device according to any of claim 1 to 8, ***wherein*** said housing (21) comprises a bottom side, said bottom side including a sensor, said sensor being configured to come in contact with the body portion.

10. Device according to any of claim 1 to 9, ***wherein*** said docking station (11) comprises a bottom wall (111) configured to come in contact with the skin, framed by two lateral walls (112, 113) comprising the second connection means (15), said bottom wall (111) and lateral walls (112, 113) forming a reception site configured to receive the control unit (20).

11. Device according to claims 9 and 10, ***wherein*** said docking station (11) comprises a through hole (17) in the bottom wall (111) of the docking station (11), said through hole (17) being configured to allow insertion of said sensor (25).

12. Device according to any of claim 1 to 11, wherein the pumping means (23) include tubing connections (50), said tubing connections (50) being made in one piece with the housing (21).

13. Device according to any of claim 1 to 12, wherein the device includes a calibration station (60) configured to calibrate at least one pressure sensor (28) of the control unit (20), said calibration station being positioned between the docking station (11) and the control unit (20).

14. Band device (10) intended for use in a wearable device according to any of claim 1 to 12, said band device (10) co-operating with a control unit (20), ***wherein*** said band device (10) is configured to circle the body portion, said band device (10) comprising:
- a bracelet (12) comprising a bladder (14) configured to be inflated and deflated; and
a docking station (11) configured to removably secure the control unit (20), said docking station (11) including second connection means (14) configured to co-operate with first connection means of the control unit (20), to allow said pump to inflate or deflate the bladder (14), and ***wherein*** a first extremity (101) of the bracelet (12) is connected to a first lateral portion of the docking station (11) and a second extremity (102) of the bracelet (12) is connected to a second lateral portion of the docking station, said first lateral portion and second lateral portion facing each other, at least one of the first lateral portion and second lateral portion forming a connecting portion, said connecting portion including said second connection means (15), the docking station (11) being interposed between the first extremity (101) and the second extremity (102) to close the band device (10).

15. Control unit (20) intended for use in a wearable device according to any of claim 1 to 12, said control unit (20) co-operating with a band device (10), ***wherein*** said control unit (20) comprises:
- a housing (21);
- pumping means (23) within the housing (21); and
- first connection means (22a, 22b) in fluidic connection with the pumping means (23), said first connection means (22a, 22b) being configured to allow a fluid to flow between the pumping means (23) and an external environment of the housing (21),
said control unit (21) being configured to calculate a blood pressure value using the pressure exerted by the bladder (14) on the body portion.
